# EUROPEAN PATENT APPLICATION

(11) **EP 2 637 023 A1**
(43) Date of publication of application: **11.09.2013**
(21) Application number: 12001590.4
(22) Date of filing: 08.03.2012
(51) Int. Cl.: G01N 33/68, G01N 33/74

(54) **Prediction of outcome in patients with chronic obstructive pulmonary disease**

(71) Applicant: B.R.A.H.M.S GmbH, 16761 Hennigsdorf (DE)
(72) Inventor: Giersdorf, Sven, 16761 Henningsdorf (DE); Tamm, Michael, 4031 Basel (CH); Stolz, Daiana, 4031 Basel (CH)
(74) Representative: Tomerius, Isabel

(57) **Abstract**

The present invention relates to a method for the prognosis and/or risk assessment and/or monitoring of therapy and/or management of patients with COPD the method comprising the steps of:
i) providing a sample of a bodily fluid from said patient,
ii) determining in said sample the level of at least one biomarker, selected from the group consisting of pro-adrenomedullin (proADM), pronatriuretic peptide, pro-Vasopressin (proAVP) and Procalcitonin (PCT) or fragments thereof of at least 12 amino acids in length,
iii) determining one, two or three of the BODE-index parameters body-mass index (BMI, parameter B), degree of airflow obstruction (FEV₁, parameter O), dyspnea (parameter D) and exercise capacity (parameter E),
iv) correlating said level of said at least one biomarker determined in step ii), in combination with said one, two or three BODE-index parameters determined in step iii) to the prognosis and/or risk assessment and/or monitoring of therapy and/or management of patients with COPD.

SEQUENCE LISTING

<110>  B.R.A.H.M.S. GmbH

<120>  Prediction of outcome in patients with chronic obstructive
       pulmonary disease

<130>  B.P 882 EP

<160>  16

<170>  PatentIn version 3.3

<210>  1
<211>  153
<212>  PRT
<213>  Homo sapiens

<400>  1


<210>  2
<211>  126
<212>  PRT
<213>  Homo sapiens

<400>  2

Asn Pro Met Tyr Asn Ala Val Ser Asn Ala Asp Leu Met Asp Phe Lys

1               5                   10                  15

Asn Leu Leu Asp His Leu Glu Glu Lys Met Pro Leu Glu Asp Glu Val
            20                  25                  30

Val Pro Pro Gln Val Leu Ser Glu Pro Asn Glu Glu Ala Gly Ala Ala
        35                  40                  45

Leu Ser Pro Leu Pro Glu Val Pro Pro Trp Thr Gly Glu Val Ser Pro
    50                  55                  60

Ala Gln Arg Asp Gly Gly Ala Leu Gly Arg Gly Pro Trp Asp Ser Ser
65                  70                  75                  80

Asp Arg Ser Ala Leu Leu Lys Ser Lys Leu Arg Ala Leu Leu Thr Ala
                85                  90                  95

Pro Arg Ser Leu Arg Arg Ser Ser Cys Phe Gly Gly Arg Met Asp Arg
            100                 105                 110

Ile Gly Ala Gln Ser Gly Leu Gly Cys Asn Ser Phe Arg Tyr
        115                 120                 125

<210>  3
<211>  98
<212>  PRT
<213>  Homo sapiens

<400>  3

Asn Pro Met Tyr Asn Ala Val Ser Asn Ala Asp Leu Met Asp Phe Lys
1               5                   10                  15

Asn Leu Leu Asp His Leu Glu Glu Lys Met Pro Leu Glu Asp Glu Val
            20                  25                  30

Val Pro Pro Gln Val Leu Ser Glu Pro Asn Glu Glu Ala Gly Ala Ala
        35                  40                  45

Leu Ser Pro Leu Pro Glu Val Pro Pro Trp Thr Gly Glu Val Ser Pro
    50                  55                  60

Ala Gln Arg Asp Gly Gly Ala Leu Gly Arg Gly Pro Trp Asp Ser Ser
65                  70                  75                  80

Asp Arg Ser Ala Leu Leu Lys Ser Lys Leu Arg Ala Leu Leu Thr Ala
                85                  90                  95

Pro Arg

<210>  4
<211>  38

<212>   PRT
<213>   Homo sapiens

<400>   4

Pro Glu Val Pro Pro Trp Thr Gly Glu Val Ser Pro Ala Gln Arg Asp
1               5                   10                  15

Gly Gly Ala Leu Gly Arg Gly Pro Trp Asp Ser Ser Asp Arg Ser Ala
            20                  25                  30

Leu Leu Lys Ser Lys Leu
        35

<210>   5
<211>   185
<212>   PRT
<213>   Homo sapiens

<400>   5

Met Lys Leu Val Ser Val Ala Leu Met Tyr Leu Gly Ser Leu Ala Phe
1               5                   10                  15

Leu Gly Ala Asp Thr Ala Arg Leu Asp Val Ala Ser Glu Phe Arg Lys
            20                  25                  30

Lys Trp Asn Lys Trp Ala Leu Ser Arg Gly Lys Arg Glu Leu Arg Met
        35                  40                  45

Ser Ser Ser Tyr Pro Thr Gly Leu Ala Asp Val Lys Ala Gly Pro Ala
    50                  55                  60

Gln Thr Leu Ile Arg Pro Gln Asp Met Lys Gly Ala Ser Arg Ser Pro
65                  70                  75                  80

Glu Asp Ser Ser Pro Asp Ala Ala Arg Ile Arg Val Lys Arg Tyr Arg
                85                  90                  95

Gln Ser Met Asn Asn Phe Gln Gly Leu Arg Ser Phe Gly Cys Arg Phe
            100                 105                 110

Gly Thr Cys Thr Val Gln Lys Leu Ala His Gln Ile Tyr Gln Phe Thr
        115                 120                 125

Asp Lys Asp Lys Asp Asn Val Ala Pro Arg Ser Lys Ile Ser Pro Gln
    130                 135                 140

Gly Tyr Gly Arg Arg Arg Arg Arg Ser Leu Pro Glu Ala Gly Pro Gly
145                 150                 155                 160

Arg Thr Leu Val Ser Ser Lys Pro Gln Ala His Gly Ala Pro Ala Pro
                165                 170                 175

Pro Ser Gly Ser Ala Pro His Phe Leu
            180                 185

<210> 6
<211> 164
<212> PRT
<213> Homo sapiens

<400> 6

Ala Arg Leu Asp Val Ala Ser Glu Phe Arg Lys Lys Trp Asn Lys Trp
1               5                   10                  15

Ala Leu Ser Arg Gly Lys Arg Glu Leu Arg Met Ser Ser Ser Tyr Pro
            20                  25                  30

Thr Gly Leu Ala Asp Val Lys Ala Gly Pro Ala Gln Thr Leu Ile Arg
        35                  40                  45

Pro Gln Asp Met Lys Gly Ala Ser Arg Ser Pro Glu Asp Ser Ser Pro
    50                  55                  60

Asp Ala Ala Arg Ile Arg Val Lys Arg Tyr Arg Gln Ser Met Asn Asn
65                  70                  75                  80

Phe Gln Gly Leu Arg Ser Phe Gly Cys Arg Phe Gly Thr Cys Thr Val
                85                  90                  95

Gln Lys Leu Ala His Gln Ile Tyr Gln Phe Thr Asp Lys Asp Lys Asp
            100                 105                 110

Asn Val Ala Pro Arg Ser Lys Ile Ser Pro Gln Gly Tyr Gly Arg Arg
        115                 120                 125

Arg Arg Arg Ser Leu Pro Glu Ala Gly Pro Gly Arg Thr Leu Val Ser
    130                 135                 140

Ser Lys Pro Gln Ala His Gly Ala Pro Ala Pro Pro Ser Gly Ser Ala
145                 150                 155                 160

Pro His Phe Leu

<210> 7
<211> 48
<212> PRT
<213> Homo sapiens

<400> 7

Glu Leu Arg Met Ser Ser Ser Tyr Pro Thr Gly Leu Ala Asp Val Lys
1               5                   10                  15

Ala Gly Pro Ala Gln Thr Leu Ile Arg Pro Gln Asp Met Lys Gly Ala
            20                  25                  30

Ser Arg Ser Pro Glu Asp Ser Ser Pro Asp Ala Ala Arg Ile Arg Val
        35                  40                  45

<210>  8
<211>  164
<212>  PRT
<213>  Homo sapiens

<400>  8

<210>  9
<211>  145
<212>  PRT
<213>  Homo sapiens

<400>  9

Cys Tyr Phe Gln Asn Cys Pro Arg Gly Gly Lys Arg Ala Met Ser Asp
1               5                   10                  15

Leu Glu Leu Arg Gln Cys Leu Pro Cys Gly Pro Gly Gly Lys Gly Arg
            20                  25                  30

Cys Phe Gly Pro Ser Ile Cys Cys Ala Asp Glu Leu Gly Cys Phe Val
        35                  40                  45

Gly Thr Ala Glu Ala Leu Arg Cys Gln Glu Glu Asn Tyr Leu Pro Ser
    50                  55                  60

Pro Cys Gln Ser Gly Gln Lys Ala Cys Gly Ser Gly Gly Arg Cys Ala
65                  70                  75                  80

Ala Phe Gly Val Cys Cys Asn Asp Glu Ser Cys Val Thr Glu Pro Glu
                85                  90                  95

Cys Arg Glu Gly Phe His Arg Arg Ala Arg Ala Ser Asp Arg Ser Asn
            100                 105                 110

Ala Thr Gln Leu Asp Gly Pro Ala Gly Ala Leu Leu Leu Arg Leu Val
        115                 120                 125

Gln Leu Ala Gly Ala Pro Glu Pro Phe Glu Pro Ala Gln Pro Asp Ala
    130                 135                 140

Tyr
145

<210>  10
<211>  39
<212>  PRT
<213>  Homo sapiens

<400>  10

Ala Ser Asp Arg Ser Asn Ala Thr Gln Leu Asp Gly Pro Ala Gly Ala
1               5                   10                  15

Leu Leu Leu Arg Leu Val Gln Leu Ala Gly Ala Pro Glu Pro Phe Glu
            20                  25                  30

Pro Ala Gln Pro Asp Ala Tyr
        35

<210>  11
<211>  93
<212>  PRT
<213>  Homo sapiens

<400>  11

Ala Met Ser Asp Leu Glu Leu Arg Gln Cys Leu Pro Cys Gly Pro Gly
1               5                   10                  15

Gly Lys Gly Arg Cys Phe Gly Pro Ser Ile Cys Cys Ala Asp Glu Leu
            20                  25                  30

Gly Cys Phe Val Gly Thr Ala Glu Ala Leu Arg Cys Gln Glu Glu Asn
        35                  40                  45

Tyr Leu Pro Ser Pro Cys Gln Ser Gly Gln Lys Ala Cys Gly Ser Gly
    50                  55                  60

Gly Arg Cys Ala Ala Phe Gly Val Cys Cys Asn Asp Glu Ser Cys Val
65                  70                  75                  80

Thr Glu Pro Glu Cys Arg Glu Gly Phe His Arg Arg Ala
                85                  90

<210>  12
<211>  116
<212>  PRT
<213>  Homo sapiens

<400>  12

Ala Pro Phe Arg Ser Ala Leu Glu Ser Ser Pro Ala Asp Pro Ala Thr
1               5                   10                  15

Leu Ser Glu Asp Glu Ala Arg Leu Leu Leu Ala Ala Leu Val Gln Asp
            20                  25                  30

Tyr Val Gln Met Lys Ala Ser Glu Leu Glu Gln Glu Gln Glu Arg Glu
        35                  40                  45

Gly Ser Ser Leu Asp Ser Pro Arg Ser Lys Arg Cys Gly Asn Leu Ser
    50                  55                  60

Thr Cys Met Leu Gly Thr Tyr Thr Gln Asp Phe Asn Lys Phe His Thr
65                  70                  75                  80

Phe Pro Gln Thr Ala Ile Gly Val Gly Ala Pro Gly Lys Lys Arg Asp
                85                  90                  95

Met Ser Ser Asp Leu Glu Arg Asp His Arg Pro His Val Ser Met Pro
            100                 105                 110

Gln Asn Ala Asn
        115

<210>  13
<211>  134
<212>  PRT
<213>  Homo sapiens

<400>  13

Met Asp Pro Gln Thr Ala Pro Ser Arg Ala Leu Leu Leu Leu Leu Phe
1               5                   10                  15

Leu His Leu Ala Phe Leu Gly Gly Arg Ser His Pro Leu Gly Ser Pro
            20                  25                  30

Gly Ser Ala Ser Asp Leu Glu Thr Ser Gly Leu Gln Glu Gln Arg Asn
        35                  40                  45

His Leu Gln Gly Lys Leu Ser Glu Leu Gln Val Glu Gln Thr Ser Leu
    50                  55                  60

Glu Pro Leu Gln Glu Ser Pro Arg Pro Thr Gly Val Trp Lys Ser Arg
65                  70                  75                  80

Glu Val Ala Thr Glu Gly Ile Arg Gly His Arg Lys Met Val Leu Tyr
                85                  90                  95

Thr Leu Arg Ala Pro Arg Ser Pro Lys Met Val Gln Gly Ser Gly Cys
            100                 105                 110

Phe Gly Arg Lys Met Asp Arg Ile Ser Ser Ser Ser Gly Leu Gly Cys
        115                 120                 125

Lys Val Leu Arg Arg His
    130

<210>  14
<211>  108
<212>  PRT
<213>  Homo sapiens

<400>  14

His Pro Leu Gly Ser Pro Gly Ser Ala Ser Asp Leu Glu Thr Ser Gly
1               5                   10                  15

Leu Gln Glu Gln Arg Asn His Leu Gln Gly Lys Leu Ser Glu Leu Gln
            20                  25                  30

Val Glu Gln Thr Ser Leu Glu Pro Leu Gln Glu Ser Pro Arg Pro Thr
        35                  40                  45

Gly Val Trp Lys Ser Arg Glu Val Ala Thr Glu Gly Ile Arg Gly His
    50                  55                  60

Arg Lys Met Val Leu Tyr Thr Leu Arg Ala Pro Arg Ser Pro Lys Met
65                  70                  75                  80

Val Gln Gly Ser Gly Cys Phe Gly Arg Lys Met Asp Arg Ile Ser Ser
                85                  90                  95

Ser Ser Gly Leu Gly Cys Lys Val Leu Arg Arg His
            100                 105

<210>  15

<211>  76
<212>  PRT
<213>  Homo sapiens

<400>  15

His Pro Leu Gly Ser Pro Gly Ser Ala Ser Asp Leu Glu Thr Ser Gly
1               5                   10                  15

Leu Gln Glu Gln Arg Asn His Leu Gln Gly Lys Leu Ser Glu Leu Gln
            20                  25                  30

Val Glu Gln Thr Ser Leu Glu Pro Leu Gln Glu Ser Pro Arg Pro Thr
        35                  40                  45

Gly Val Trp Lys Ser Arg Glu Val Ala Thr Glu Gly Ile Arg Gly His
    50                  55                  60

Arg Lys Met Val Leu Tyr Thr Leu Arg Ala Pro Arg
65                  70                  75

<210>  16
<211>  32
<212>  PRT
<213>  Homo sapiens

<400>  16

Ser Pro Lys Met Val Gln Gly Ser Gly Cys Phe Gly Arg Lys Met Asp
1               5                   10                  15

Arg Ile Ser Ser Ser Ser Gly Leu Gly Cys Lys Val Leu Arg Arg His
            20                  25                  30

## Description

### Field of the invention

The present invention is in the field of clinical diagnostics. Particularly, the present invention relates to the determination of the level of biomarkers in a sample derived from a bodily fluid of a patient with COPD.

### Background of the invention

Chronic obstructive pulmonary disease (COPD), a common preventable and treatable disease, is characterized by persistent airflow limitation that is usually progressive and associated with an enhanced chronic inflammatory response in the airways and the lung to noxious particles or gases. Symptoms of COPD include dyspnea, chronic cough and chronic sputum production. Spirometry is required to make a clinical diagnosis of COPD. The presence of a postbronchodilator FEV₁/FVC (forced expiratory volume in one second/ forced vital capacity) < 0.70 confirms the presence of persistent airflow limitation and thus COPD.

The prevalence of clinically relevant COPD is about 4-6% in the European adult population with estimated numbers of COPD patients of 3.0 million in the UK, 2.7 million in Germany and 2.6 million in France. In the United States COPD affects approximately 16 million adults. Moreover, COPD is one of the fastest growing causes of morbidity and mortality (Pauwels and Rabe 2004. Lancet 364:613-620*;* Sullivan et al. 2000. Chest 117:5S-9S*;* Lopez et al. 2006. Lancet 367:1747-1757). It is the fifth leading cause of death worldwide and further increases in its prevalence and mortality are expected in the coming decades (Pauwels and Rabe 2004. Lancet 364: 613-620*).*

Exacerbations of chronic obstructive pulmonary disease (episodes of acute worsening of symptoms) are responsible for more than 2.4% of all acute medical admissions and constitute the most important direct healthcare costs associated with COPD (Donaldson et al. 2006. Thorax 61:164-168*;* Halbert et al. 2006. Eur Respir J 28:523-532*;* Lindenauer et al. 2006. Ann Intern Med 144:894-903). In the USA, the mean cost of hospital admission by COPD in a cohort of patients with severe COPD was estimated to be US$ 7,100 (Halbert et al. 2006. Eur Respir J 28:523-532*;* Connors et al. 1998. Am J Respir Crit Care Med 154:959-967). Exacerbations are now recognized as important events in the natural course of disease progression (Celli et al. 2007. Eur Respir J 29:1224-1238*).* Several clinical characteristics have been extensively validated as prognostic factors of morbidity and mortality in AECOPD (Connors et al. 1998. Am J Respir Crit Care Med 154:959-967*;* Antonelli Incalzi et al. 1997. Eur Respir J 10:2794-2800*;* Fuso et al. 1995. Am J Med 98:272-277*;* Gunen et al. 2005. Eur Respir J 26:234-24*;* Almagro et al. 2006. Respiration 73:311-317).
Although of epidemiological interest, the predictive value of clinical parameters vary in the different studies and the majority of them do not allow precise individual risk-assessment (Antonelli Incalzi et al. 1997. Eur Respir J 10:2794-2800*;* Yohannes et al. 2005. Age Ageing 34:491-496*;* Almagro et al. 2006. Respiration 73:311-317).

The goals of COPD assessment are to determine the severity of the disease, its impact on patient's health status, and the risk of future events (exacerbations, hospital admission, death) in order to guide therapy.

The degree of airflow limitation is assessed using spirometry (pulmonary function tests): The volume in a one-second forced exhalation is called the forced expiratory volume in one second (FEV₁), measured in liters. The total exhaled breath is called the forced vital capacity (FVC), also measured in liters. In people with normal lung function, FEV₁ is at least 70% of FVC. The GOLD (Global Initiative for Chronic Obstructive Lung Disease) COPD classification system is then used to describe the severity of the obstruction or airflow limitation. The worse a person's airflow limitation is, the lower their FEV₁. As COPD progresses, FEV₁ tends to decline. GOLD COPD staging uses four categories of severity for COPD, based on the value of FEV₁ which is summarized in Table 1.

The BODE index (body-mass index (B), the degree of airflow obstruction (O), measured by lung function testing and dyspnea (D), and exercise capacity (E), measured by the six-minute-walk test), has been recently established and was shown to be a tool for the prognosis of mortality (Celli et al. 2004. N Engl J Med; 350:1005-1012) and hospitalization for COPD (Ong et al. 2005. Chest 128:3810-381) in patients with COPD. However, the determination of this index is cumbersome, as it requires a 6-minute walk test (6MWT) in stable state of the disease and is also not suitable for acute exacerbations. Thus, there has been increasing interest in using other parameters, including pulmonary biomarkers to monitor disease severity in patients with COPD (Barnes et al. 2006. Am J Respir Crit Care Med 174:6-14). Several circulating biomarkers, including TNF-α, IL-6, C-reactive protein, endothelin-1 and procalcitonin are increased during exacerbations, potentially reflecting spillover of local airway inflammation into the circulation (Hurst et al. 2006. Am J Respir Crit Care Med 174:867-874*;* Pinto-Plata et al. 2007. Chest 131:37-43*;* Roland et al. 2001. Thorax 56:30-35*;* Stolz et al. 2007. Chest 131:9-19*).*

Systemic markers have also recently gained general interest as means of determining disease severity and prognosis in stable COPD (Dahl et al. 2007. Am J Respir Crit Care Med 175:250- 255*;* de Torres et al. 2006. Eur Respir J 27:902-907*;* Man et al. 2006. Thorax 61:849-853)*.* However, there is still scarce information about how current biomarkers relate to significant clinical outcomes, particularly survival, during and outside AECOPD and whether any systemic biomarker is able to replace the BODE index in regard to prognosis in stable COPD (Barnes et al. 2006. Am J Respir Crit Care Med 174:6-14 Franciosi et al. 2006. Respir Res 7:74*;* Bhowmik et al. 2000. Thorax 55:114-120*;* Wedzicha et al. 2000. Thromb Haemost 84:210-215*;* Dev et al. 1998. Respir Med 92:664-667).

It has recently been shown that the biomarkers copeptin, mid-regional pro-adrenomedullin (MR-proADM) and mid-regional pro-atrial natriuretic peptide (MR-proANP) might be helpful in the prognosis of patients with severe exacerbations of COPD requiring hospitalization (Stolz et al. 2007. Chest 131:1058-1067*;* Stolz et al. 2008. Chest 134:263-272*;* Bernasconi et al. 2011. Chest 140:91-99)*.* However, the prognostic value of such biomarkers in stable conditions is still unknown and a risk stratification strategy for ambulatory patients is missing.

A primary object of the present invention is the provision of a method which allows an easy and reliable prognosis and/or risk assessment and/or monitoring of therapy and/or management of patients with COPD.
A further object is to provide a method which allows a prognosis and/or risk assessment and/or monitoring of therapy and/or management of patients with COPD with minimum inconvenience for said patient.
A still further object is to provide a method wherein at least part of the conventional determination of the BODE-index parameters can be replaced, preferably thus reducing the burden on the patient.

### Summary of the invention

Subject of the invention is a method for the prognosis and/or risk assessment and/or monitoring of therapy and/or management of patients with COPD the method comprising the steps of:
i) providing a sample of a bodily fluid from said patient,
ii) determining in said sample the level of at least one biomarker, selected from the group consisting of pro-adrenomedullin (proADM), pro-natriuretic peptide, pro-Vasopressin (proAVP) and Procalcitonin (PCT) or fragments thereof of at least 12 amino acids in length,
iii) determining one, two, or three of the BODE-index parameters body-mass index (BMI, parameter B), degree of airflow obstruction (FEV₁, parameter O), dyspnea (parameter D) and exercise capacity (parameter E),
iv) correlating said level of said at least one biomarker, determined in step ii), in combination with said one, two or three BODE-index parameters determined in step iii) to the prognosis and/or risk assessment and/or monitoring of therapy and/or management of patients with COPD.

Preferred method variants are described in the dependent claims.

### Description of drawings

**Fig. 1****:** Box-and-whisker plot of MR-proADM values for the prediction of death in patients with COPD within 2 years.
**Fig. 2****:** Box-and-whisker plot of MR-proANP values for the prediction of death in patients with COPD within 2 years.
**Fig. 3****:** Box-and-whisker plot of Copeptin values for the prediction of death in patients with COPD within 2 years.
**Fig. 4****:** Box-and-whisker plot of PCT values for the prediction of death in patients with COPD within 2 years.
**Fig. 5****:** ROC plot for MR-proADM for the prediction of death in patients with COPD within 2 years (AUC = 0.632).
**Fig. 6****:** ROC plot for MR-proANP for the prediction of death in patients with COPD within 2 years (AUC = 0.611).
**Fig. 7****:** ROC plot for Copeptin for the prediction of death in patients with COPD within 2 years (AUC = 0.605).
**Fig. 8****:** ROC plot for PCT for the prediction of death in patients with COPD within 2 years (AUC = 0.604).
**Fig. 9****:** Kaplan-Meier survival curves (death within 2 years) by quartiles of MR-proADM for patients with COPD.
**Fig. 10****:** Kaplan-Meier survival curves (death within 2 years) by quartiles of MR-proANP for patients with COPD.
**Fig. 11****:** Kaplan-Meier survival curves (death within 2 years) by quartiles of Copeptin for patients with COPD.
**Fig. 12****:** Kaplan-Meier survival curves (death within 2 years) by quartiles of PCT for patients with COPD.
**Fig. 13****:** ROC plot for MR-proADM in combination with BODE for the prediction of death in patients with COPD within 2 years (AUC = 0.750).
**Fig. 14****:** ROC plot for MR-proADM in combination with BOD for the prediction of death in patients with COPD within 2 years (AUC = 0.743).
**Fig. 15****:** ROC plot for MR-proADM in combination with BD for the prediction of death in patients with COPD within 2 years (AUC = 0.756).
**Fig. 16****:** ROC plot for MR-proANP in combination with BODE for the prediction of death in patients with COPD within 2 years (AUC = 0.736).
**Fig. 17****:** ROC plot for MR-proANP in combination with BOD for the prediction of death in patients with COPD within 2 years (AUC = 0.727).
**Fig. 18****:** ROC plot for MR-proANP in combination with BD for the prediction of death in patients with COPD within 2 years (AUC = 0.741).
**Fig. 19****:** ROC plot for Copeptin in combination with BODE for the prediction of death in patients with COPD within 2 years (AUC = 0.710).
**Fig. 20****:** ROC plot for Copeptin in combination with BOD for the prediction of death in patients with COPD within 2 years (AUC = 0.703).
**Fig. 21****:** ROC plot for Copeptin in combination with BD for the prediction of death in patients with COPD within 2 years (AUC = 0.724).
**Fig. 22****:** ROC plot for PCT in combination with BODE for the prediction of death in patients with COPD within 2 years (AUC = 0.698).
**Fig. 23****:** ROC plot for PCT in combination with BOD for the prediction of death in patients with COPD within 2 years (AUC = 0.678).
**Fig. 24****:** ROC plot for PCT in combination with BD for the prediction of death in patients with COPD within 2 years (AUC = 0.706).
**Fig. 25****:** Kaplan-Meier survival curves (death within 2 years) for the combination of BODE and MR-proADM (applied as categorical variable using cut-offs and subsequent scoring as indicated in table 5) by dividing the patients with COPD into risk groups depending on the combined score.
**Fig. 26****:** Kaplan-Meier survival curves (death within 2 years) for the combination of BOD and MR-proADM (applied as categorical variable using cut-offs and subsequent scoring as indicated in table 5) by dividing the patients with COPD into risk groups depending on the combined score.
**Fig. 27****:** Kaplan-Meier survival curves (death within 2 years) for the combination of BD and MR-proADM (applied as categorical variable using cut-offs and subsequent scoring as indicated in table 5) by dividing the patients with COPD into risk groups depending on the combined score.
**Fig. 28****:** Kaplan-Meier survival curves (death within 2 years) for the combination of BODE and MR-proANP (applied as categorical variable using cut-offs and subsequent scoring as indicated in table 5) by dividing the patients with COPD into risk groups depending on the combined score.
**Fig. 29****:** Kaplan-Meier survival curves (death within 2 years) for the combination of BOD and MR-proANP (applied as categorical variable using cut-offs and subsequent scoring as indicated in table 5) by dividing the patients with COPD into risk groups depending on the combined score.
**Fig. 30****:** Kaplan-Meier survival curves (death within 2 years) for the combination of BD and MR-proANP (applied as categorical variable using cut-offs and subsequent scoring as indicated in table 5) by dividing the patients with COPD into risk groups depending on the combined score.
**Fig. 31****:** Kaplan-Meier survival curves (death within 2 years) for the combination of BODE and Copeptin (applied as categorical variable using cut-offs and subsequent scoring as indicated in table 5) by dividing the patients with COPD into risk groups depending on the combined score.
**Fig. 32****:** Kaplan-Meier survival curves (death within 2 years) for the combination of BOD and Copeptin (applied as categorical variable using cut-offs and subsequent scoring as indicated in table 5) by dividing the patients with COPD into risk groups depending on the combined score.
**Fig. 33****:** Kaplan-Meier survival curves (death within 2 years) for the combination of BD and Copeptin (applied as categorical variable using cut-offs and subsequent scoring as indicated in table 5) by dividing the patients with COPD into risk groups depending on the combined score.
**Fig. 34****:** Kaplan-Meier survival curves (death within 2 years) for the combination of BODE and PCT (applied as categorical variable using cut-offs and subsequent scoring as indicated in table 5) by dividing the patients with COPD into risk groups depending on the combined score.
**Fig. 35****:** Kaplan-Meier survival curves (death within 2 years) for the combination of BOD and PCT (applied as categorical variable using cut-offs and subsequent scoring as indicated in table 5) by dividing the patients with COPD into risk groups depending on the combined score.
**Fig. 36****:** Kaplan-Meier survival curves (death within 2 years) for the combination of BD and PCT (applied as categorical variable using cut-offs and subsequent scoring as indicated in table 5) by dividing the patients with COPD into risk groups depending on the combined score.

### Detailed description of the invention

Subject of the invention is a method for the prognosis and/or risk assessment and/or monitoring of therapy and/or management of patients with COPD the method comprising the steps of:
i) providing a sample of a bodily fluid from said patient,
ii) determining in said sample the level of at least one biomarker, selected from the group consisting of pro-adrenomedullin (proADM), pro-natriuretic peptide, pro-Vasopressin (proAVP) and Procalcitonin (PCT) or fragments thereof of at least 12 amino acids in length,
iii) determining one, two or three of the BODE-index parameters body-mass index (BMI, parameter B), degree of airflow obstruction (FEV₁, parameter O), dyspnea (parameter D) and exercise capacity (parameter E),
iv) correlating said level of said at least one biomarker determined in step ii), in combination with said one, two or three BODE-index parameters determined in step iii) to the prognosis and/or risk assessment and/or monitoring of therapy and/or management of patients with COPD.

The term "combination" is defined as a possible selection of a certain number of parameters and the arrangement of these parameters into specified groups using a mathematical algorithm.

The term "parameter" is used in the present invention as a characteristic, a feature or a measurable factor that can help in defining a particular system, e.g. a biomarker (for example copeptin or PCT), a descriptive variable (for example age, gender, BMI) or a clinical variable (for example FEV₁).

In a preferred embodiment, said level of said at least one biomarker or fragments thereof of at least 12 amino acids in length is used in combination with either the BODE-index parameters body-mass index (BMI, parameter B), degree of airflow obstruction (FEV₁, parameter O), and dyspnea (parameter D) or the BODE-index parameters body-mass index (BMI, parameter B) and dyspnea (parameter D).

In another preferred embodiment, the level of at least one biomarker, selected from the group consisting of proADM, pro-natriuretic peptides, proAVP and PCT or fragments thereof of at least 12 amino acids in length and said one, two or three BODE-index parameters can be combined as continuous or categorical variables.

In a preferred embodiment the level of at least one biomarker, selected from the group consisting of proADM, pro-natriuretic peptides, proAVP and PCT or fragments thereof of at least 12 amino acids in length and said one, two or three BODE-index parameters can be combined in a score. For example, the biomarker levels can be dichotomized or categorized by using one or more cut-off values to form a binary or categorical variable. The respective variable or score can then be added to a score, e.g. retrieved from the index parameters BOD or BD, to a combined score.

In a particular embodiment of the inventive methods the level of at least one biomarker, selected from the group consisting of proADM, pro-natriuretic peptides, proAVP and PCT or fragments thereof of at least 12 amino acids in length and said one, two or three BODE-index parameters are differently weighted.

In another preferred embodiment the prognosis and/or risk assessment relates to the risk of mortality and patients are stratified into potential survivors and potential non-survivors.

In an especially preferred embodiment the prognosis and/or risk assessment relates to the risk assessment of mortality within 5 years, more preferred within 4 year, even more preferred within 3 years, even more preferred within 2 years, even more preferred within 1 year, most preferred within 6 months.

In another preferred embodiment the prognosis and/or risk assessment relates to the risk of the occurrence of acute exacerbations and patients are stratified into either a group of patients likely getting an acute exacerbation or into a group of patients which do not likely get an acute exacerbation.

In an especially preferred embodiment the prognosis and/or risk assessment relates to the risk assessment of getting an acute exacerbation within 2 years, more preferred within 1 year, even more preferred within 6 months, even more preferred within 3 months, even more preferred within 90 days, even more preferred within 1 month, even more preferred within 30 days, even more preferred within 14 days, most preferred within 7 days.

Chronic obstructive pulmonary disease (COPD) is diagnosed according to the GOLD guidelines (FEV1/FVC ratio below 70% and an absolute reduction of FEV1 below 80% of the predicted value).

According to the present invention a patient diagnosed with COPD may be in the stable or unstable (acute exacerbated) state of the disease.

Acute exacerbation of COPD is defined as "an event in the natural course of the disease characterized by a change in the patient's baseline dyspnea, cough, and/or sputum that is beyond normal day-to-day variations, is acute in onset, and may warrant a change in regular medication in a patient with underlying COPD" (Rabe et al. 2007. Am J Respir Crit Care Med 176:532-555).

Severity of COPD is graded according to GOLD criteria, using the postbronchodilator FEV₁ % predicted. An FEV₁ between 50 and 80% of the predicted value defines a moderate COPD (GOLD II), an FEV₁ between 30% and 50% a severe COPD (GOLD III), and an FEV₁ below 30% a very severe COPD (GOLD IV), see Table 1. The volume in a one-second forced exhalation is called the forced expiratory volume in one second (FEV₁), measured in liters.

The BODE is a multidimensional index designed to assess clinical risk in people with COPD (Celli et al. 2004. N Engl J Med 350:1005-1012*).* It combines four variables into a single score: (B) body mass index, (O) airflow obstruction measured by the forced expiratory volume in one second (FEV₁), (D) dyspnea measured by the modified Medical Research Council (MRC) scale, and (E) exercise capacity measured by the 6-minute walk distance (6MWD). Each component is graded and a score out of 10 is obtained (Table 2), with higher scores indicating greater risk. The BODE index reflects the impact of both pulmonary and extrapulmonary factors on prognosis and survival in COPD.

In the present invention, the term "prognosis" denotes a prediction of how a subject's (e.g. a patient's) medical condition will progress. This may include an estimation of the chance of recovery or the chance of an adverse outcome for said subject.

In the present invention, the term "risk assessment" denotes an assignment of a probability to experience certain adverse events to an individual. Hereby, the individual may preferably be accounted to a certain risk category, wherein categories comprise for instance high risk versus low risk, or risk categories based on numeral values, such as risk category 1, 2, 3, etc.

The term "therapy monitoring" in the context of the present invention refers to the control and/or adjustment of a therapeutic treatment of said patient.

The term "patient management" in the context of the present invention refers to:
● the decision for admission to hospital or intensive care unit,
● the decision for relocation of the patient to a specialized hospital or a specialized hospital unit,
● the evaluation for an early discharge from the intensive care unit or hospital,
● the allocation of resources (e.g. physician and/or nursing staff, diagnostics, therapeutics),
● the decision on therapeutic treatment.

The term "correlating", as used herein in reference to the use of diagnostic and prognostic marker(s), refers to comparing the presence or level of the marker(s) in a patient to its presence or amount in persons known to suffer from, or known to be at risk of, a given condition. A marker level in a patient sample can be compared to a level known to be associated with a specific diagnosis. The sample's marker level is said to have been correlated with a diagnosis; that is, the skilled artisan can use the marker level to determine whether the patient suffers from a specific type of a disease, and respond accordingly. Alternatively, the sample's marker level can be compared to a marker level known to be associated with a good outcome (e.g. the absence of disease etc.). In preferred embodiments, a panel of marker levels is correlated to a global probability or a particular outcome.

Threshold levels can be obtained for instance from a Kaplan-Meier analysis, where the occurrence of a disease or the probability of a serious condition and/or death is correlated with the e.g. quartiles of the respective markers in the population. According to this analysis, subjects with marker levels above the 75th percentile have a significantly increased risk for getting the diseases according to the invention. This result is further supported by Cox regression analysis with adjustment for classical risk factors. The highest quartile versus all other subjects is highly significantly associated with increased risk for getting a disease or the probability of a serious condition and/or death according to the invention.
Other preferred cut-off values are for instance the 90th, 95th or 99th percentile of a reference population. By using a higher percentile than the 75th percentile, one reduces the number of false positive subjects identified, but one might miss to identify subjects, who are at moderate, albeit still increased risk. Thus, one might adapt the cut-off value depending on whether it is considered more appropriate to identify most of the subjects at risk at the expense of also identifying "false positives", or whether it is considered more appropriate to identify mainly the subjects at high risk at the expense of missing several subjects at moderate risk.
Other mathematical possibilities to calculate an individual's risk by using the individual's marker level value and other prognostic laboratory and clinical parameters are for instance the NRI (Net Reclassification Index) or the IDI (Integrated Discrimination Index). The indices can be calculated according to Pencina (Pencina MJ, et al.: Evaluating the added predictive ability of a new marker: from area under the ROC curve to reclassification and beyond. Stat Med. 2008;27:157-172).

As mentioned herein in the context of marker peptides and precursors thereof the term "fragment" refers to smaller proteins or peptides derivable from larger proteins or peptides, which hence comprise a partial sequence of the larger protein or peptide. Said fragments are derivable from the larger proteins or peptides by saponification of one or more of its peptide bonds. "Fragments" of the marker peptides proADM, pro-natriuretic peptide, proAVP, and PCT preferably relate to fragments of at least 12 amino acids in length. Such fragments are preferably detectable with immunological assays as described herein.

Pro-natriuretic peptides are selected from the group consisting of pro-atrial natriuretic peptide (proANP) and pro-brain natriuretic peptide (proBNP) or fragments thereof of at least 12 amino acids in length.

The sequence of the 153 amino acid pre-proANP is shown in SEQ ID NO:1. Upon cleavage of an N-terminal signal peptide (25 amino acids) and the two C-terminal amino acids, pro-ANP (SEQ ID NO:2) is released. This prohormone is cleaved into the mature 28 amino acid peptide ANP, also known as ANP (1-28) or α-ANP, and the amino terminal proANP fragment (1-98) (NT-proANP, SEQ ID NO:3). Mid-regional proANP (MR-proANP) is defined as NT-proANP or any fragments thereof comprising at least amino acid residues 53-90 (SEQ ID NO:4) of proANP.

In a preferred embodiment of the method according to the invention the level of the proANP precursor fragment, MR-proANP, is determined.

The amino acid sequence of the precursor peptide of Adrenomedullin (pre-pro-Adrenomedullin) is given in SEQ ID NO:5. Pro-Adrenomedullin relates to amino acid residues 22 to 185 of the sequence of pre-pro-Adrenomedullin. The amino acid sequence of pro-Adrenomedullin (proADM) is given in SEQ ID NO:6. MR-pro-Adrenomedullin (MR-proADM) relates to amino acid residues 45-92 of pre-proADM. The amino acid sequence of MR-proADM is provided in SEQ ID NO:7.

In another preferred embodiment of the method according to the invention the level of the proADM precursor fragment, MR-proADM, is determined.

The sequence of the 164 amino acid precursor peptide of Vasopressin (pre-pro-Vasopressin) is given in SEQ ID NO:8. Pro-Vasopressin relates to the amino acid residues 19 to 164 of the sequence of pre-pro-Vasopressin. The amino acid sequence of pro-Vasopressin is given in SEQ ID NO:9. Pro-Vasopressin is cleaved into mature Vasopressin, Neurophysin II and C-terminal proVasopressin (CT-proAVP or Copeptin). Copeptin relates to amino acid residues 126 to 164 of pre-pro-Vasopressin. The amino acid sequence of Copeptin is provided in SEQ ID NO:10. Neurophysin II comprises the amino acid residues 32 to 124 of pre-pro-Vasopressin and its sequence is shown in SEQ ID NO:11.
In another preferred embodiment of the method according to the invention the level of the proAVP precursor fragment, Copeptin, is determined.

Procalcitonin is a precursor of calcitonin and katacalcin. The amino acid sequence of PCT 1-116 is given in SEQ ID NO:12.

The sequence of the 134 amino acid precursor peptide of brain natriuretic peptide (pre-pro-BNP) is given in SEQ ID NO:13. Pro-BNP relates to amino acid residues 27 to 134 of pre-pro-BNP. The sequence of pro-BNP is shown in SEQ ID NO:14. Pro-BNP is cleaved into N-terminal pro-BNP (NT-pro-BNP) and mature BNP. NT-pro-BNP comprises the amino acid residues 27 to 102 and its sequence is shown in SEQ ID NO:15. The SEQ ID NO:16 shows the sequence of BNP comprising the amino acid residues 103 to 134 of the pre-pro-BNP peptide.

In a preferred embodiment of the method according to the invention the level of the proBNP precursor fragments, NT-proBNP or BNP, is determined.

In another preferred embodiment of the method according to the invention the level of PCT consisting of amino acids 1 to 116 or 2 to 116 or 3 to 116 is determined.

In another preferred embodiment of the invention cut-offs values can be defined for the biomarkers when used as categorized variables. For example, two different cut-off values resulting in a score of 0, 2 or 4 can be defined for the respective biomarker.

Preferably the cut-off values for MR-proADM are between 0.5 and 2 nmol/L, more preferred between 0.5 and 1 nmol/L, most preferred between 0.5 and 0.8 nmol/L. In an especially preferred embodiment of the invention the cut-off values are 0.5 and 0.8 nmol/L.

Preferably the cut-off values for MR-proANP are between 50 and 250 pmol/L, more preferred between 50 and 200 pmol/L, most preferred between 50 and 140 pmol/L. In an especially preferred embodiment of the invention the cut-off values are 50 and 140 pmol/L.

Preferably the cut-off values for Copeptin are between 2 and 30 pmol/L, more preferred between 2 and 20 pmol/L, most preferred between 2 and 14 pmol/L. In an especially preferred embodiment of the invention the cut-off values are 2 and 14 pmol/L.

Preferably the cut-off values for PCT are between 0.07 and 0.5 ng/mL, more preferred between 0.07 and 0.25 ng/mL, even more preferred between 0.07 and 0.2 ng/mL, most preferred between 0.07 and 0.1 ng/mL. In an especially preferred embodiment of the invention the cut-off values are 0.07 and 0.1 ng/mL.

The above mentioned cut-off values might be different in other assays, if these have been calibrated differently from the assay systems used in the present invention. Therefore the above mentioned cut-off values shall apply for such differently calibrated assays accordingly, taking into account the differences in calibration. One possibility of quantifying the difference in calibration is a method comparison analysis (correlation) of the assay in question (e.g. a PCT assay) with the respective biomarker assay used in the present invention (e.g. BRAHMS KRYPTOR PCT sensitive) by measuring the respective biomarker (e.g. PCT) in samples using both methods. Another possibility is to determine with the assay in question, given this test has sufficient analytical sensitivity, the median biomarker level of a representative normal population, compare results with the median biomarker levels as described in the literature (e.g. in EP 09011073.5 for PCT - "Procalcitonin for the prognosis of adverse events in the asymptomatic population") and recalculate the calibration based on the difference obtained by this comparison. With the calibration used in the present invention, samples from normal (healthy) subjects have been measured: median (interquartile range) plasma procalcitonin was 0.018 (0.015 - 0.022) ng/ml in men and 0.014 (0.012 - 0.017) ng/ml in women (Abbasi et al. 2010. JCEM 95:E26-E31), median (range) plasma MR-proADM was 0.41 *(**0.23 - 0.64) nmol*/*L (*Smith et al. 2009. Clin Chem 55:1593-1595), median (interquartile range) copeptin concentration was 4.7 (2.9 - 7.5) pmol/L, with significantly higher concentration in males (6.2 (4.1-9.5) pmol/L) than in females (3.6 (2.4-5.5) pmol/l) (Meijer et al. 2010. Kidney Int 77:29-36), and median (interquartile range) MR-proANP concentration was 66 (51 - 86) pmol/L (Melander et al. 2009. JAMA 302:49-57).

The term "score" in the context of the present invention refers to a rating, expressed numerically, based on the specific achievement or the degree to which certain qualities or conditions (e.g. the level of biomarker or said BODE-index parameters) are present in said patient.

The term "level" in the context of the present invention relates to the concentration (preferably expressed as weight/volume; w/v) of marker peptides taken from a sample of a patient. As mentioned herein, an "assay" or "diagnostic assay" can be of any type applied in the field of diagnostics. Such an assay may be based on the binding of an analyte to be detected to one or more capture probes with a certain affinity. Concerning the interaction between capture molecules and target molecules or molecules of interest, the affinity constant is preferably greater than 10⁸ M⁻¹.

In the context of the present invention, "capture molecules" are molecules which may be used to bind target molecules or molecules of interest, i.e. analytes (e.g. in the context of the present invention the cardiovascular peptide(s)), from a sample. Capture molecules must thus be shaped adequately, both spatially and in terms of surface features, such as surface charge, hydrophobicity, hydrophilicity, presence or absence of Lewis donors and/or acceptors, to specifically bind the target molecules or molecules of interest. Hereby, the binding may for instance be mediated by ionic, van-der-Waals, pi-pi, sigma-pi, hydrophobic or hydrogen bond interactions or a combination of two or more of the aforementioned interactions between the capture molecules and the target molecules or molecules of interest. In the context of the present invention, capture molecules may for instance be selected from the group comprising a nucleic acid molecule, a carbohydrate molecule, a PNA molecule, a protein, an antibody, a peptide or a glycoprotein. Preferably, the capture molecules are antibodies, including fragments thereof with sufficient affinity to a target or molecule of interest, and including recombinant antibodies or recombinant antibody fragments, as well as chemically and/or biochemically modified derivatives of said antibodies or fragments derived from the variant chain with a length of at least 12 amino acids thereof.

The preferred detection methods comprise immunoassays in various formats such as for instance radioimmunoassay (RIA), chemiluminescence- and fluorescence- immunoassays, Enzyme-linked immunoassays (ELISA), Luminex-based bead arrays, protein microarray assays, and rapid test formats such as for instance immunochromatographic strip tests.
The assays can be homogenous or heterogeneous assays, competitive and non-competitive assays. In a particularly preferred embodiment, the assay is in the form of a sandwich assay, which is a non-competitive immunoassay, wherein the molecule to be detected and/or quantified is bound to a first antibody and to a second antibody. The first antibody may be bound to a solid phase, e.g. a bead, a surface of a well or other container, a chip or a strip, and the second antibody is an antibody which is labeled, e.g. with a dye, with a radioisotope, or a reactive or catalytically active moiety. The amount of labeled antibody bound to the analyte is then measured by an appropriate method. The general composition and procedures involved with "sandwich assays" are well-established and known to the skilled person (The Immunoassay Handbook, Ed. David Wild, Elsevier LTD, Oxford; 3rd ed. (May 2005), ISBN-13: 978-0080445267*;* Hultschig C et al., Curr Opin Chem Biol. 2006 Feb;10(1):4-10. PMID: 16376134*,* incorporated herein by reference).

In a particularly preferred embodiment the assay comprises two capture molecules, preferably antibodies, which are both present as dispersions in a liquid reaction mixture, wherein a first labelling component is attached to the first capture molecule, wherein said first labelling component is part of a labelling system based on fluorescence- or chemiluminescence-quenching or amplification, and a second labelling component of said marking system is attached to the second capture molecule, so that upon binding of both capture molecules to the analyte a measurable signal is generated that allows for the detection of the formed sandwich complexes in the solution comprising the sample.

Even more preferred, said labelling system comprises rare earth cryptates or rare earth chelates in combination with a fluorescence dye or chemiluminescence dye, in particular a dye of the cyanine type. In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, such as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethoxy-fluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidium-bromide, Acridinium dyes, Carbazol dyes, Phenoxazine dyes, Porphyrine dyes, Polymethin dyes, and the like.

In the context of the present invention, chemiluminescence based assays comprise the use of dyes, based on the physical principles described for chemiluminescent materials in Kirk-Othmer, Encyclopedia of chemical technology, 4th ed., executive editor, J. I. Kroschwitz; editor, M. Howe-Grant, John Wiley & Sons, 1993, vol.15, p. 518-562*,* incorporated herein by reference, including citations on pages 551-562. Preferred chemiluminescent dyes are acri-diniumesters.

The term "sample" as used herein refers to a sample of bodily fluid obtained for the purpose of diagnosis, prognosis, or evaluation of a subject of interest, such as a patient. Preferred test samples include blood, serum, plasma, cerebrospinal fluid, urine, saliva, sputum, and pleural effusions. In addition, one of skill in the art would realize that some test samples would be more readily analyzed following a fractionation or purification procedure, for example, separation of whole blood into serum or plasma components.

Thus, in a preferred embodiment of the invention the sample is selected from the group comprising a blood sample, a serum sample, a plasma sample, a cerebrospinal fluid sample, a saliva sample and a urine sample or an extract of any of the aforementioned samples. Preferably, the sample is a blood sample, most preferably a serum sample or a plasma sample.

### Examples

### Marker Measurements

MR-proANP was detected using a novel fully automated sandwich immunoassay system on the B.R.A.H.M.S KRYPTOR (B.R.A.H.M.S GmbH, Hennigsdorf/ Berlin, Germany). This random access analyzer employs the sensitive Time Resolved Amplified Cryptate Emission (TRACE) technology, based on a non-radioactive-transfer between 2 fluorophores, europium cryptate and XL665. The automated assay is based essentially on the sandwich chemiluminescence assay which is described in detail elsewhere (Morgenthaler et al. 2004. Clin Chem 50:234-6), and which was used in other studies (Khan et al. 2008. J Am Coll Cardiol 51:1857-64*;* Gegenhuber et al. 2006. Clin Chem 52: 827-31). For MR-proANP detection, 14µl of patients' serum were incubated for 14 min. The measuring range was 0-10000 pmol/L, the limit of detection 2.1 pmol/L, and the limit of quantification 4.5 pmol/L. The intra assay CV was 1.2 % and the inter laboratory CV 5.4 %. This assay uses the same antibody pair as the reference assay (Morgenthaler et al. 2004. Clin Chem 50: 234-6), and the correlation between the two assay systems was r=0.99.

MR-proADM is detected using a novel fully automated sandwich immunoassay system on the B.R.A.H.M.S KRYPTOR (B.R.A.H.M.S GmbH, Hennigsdorf/ Berlin, Germany) (Caruhel et al. 2009. Clin Biochem 42:725-8). This random access analyzer employs the sensitive Time Resolved Amplified Cryptate Emission (TRACE) technology, based on a non-radioactive-transfer between 2 fluorophores, europium cryptate and XL665. This automated assay is based essentially on the sandwich chemiluminescence assay which is described in detail elsewhere (Morgenthaler et al. 2005 Clin Chem 51:1823-9), and which was used in other studies (Khan et al. 2007. J Am Coll Cardiol 49: 1525-32*;* Gegenhuber et al. 2007. J Card Fail 13:42-9)*.* For MR-proADM detection, 26 µl serum is incubated for 29 min. The measuring range was 0-100 nmol/L, the limit of detection and limit of quantification were 0.05 and 0.23 nmol/L, respectively. The intra assay CV is 1.9 % and the inter laboratory CV is 9.8 %. This assay uses the same antibody pair as described in detail elsewhere (Morgenthaler et al. 2005. Clin Chem 51: 1823-9)*,* and the correlation between the two assay systems is r=0.99.

Copeptin levels were measured with a chemiluminescence sandwich immunoassay with a lower detection limit of 1.7 pmol/L (Morgenthaler et al. 2006. Clin Chem 52:112-9)***.*** In 359 healthy individuals (153 men and 206 women) median Copeptin levels were 4.2 pmol/L ranging from 1.0-13.8 pmol/L. Median concentrations of Copeptin differed significantly between male and female. There was no correlation between Copeptin levels and age. The inter laboratory CV was <20% and the intra assay CV was <10% for samples > 2.25 pmol/L.

PCT was measured using an ultrasensitive commercially available test system with a functional assay sensitivity of 0.007 ng/mL as described in Morgenthaler *et al. (*Morgenthaler et al. 2002. Clin Chem 48:788-790).

### Data Analysis

Descriptive analyses were performed to summarize the baseline characteristics of the study population. Descriptive statistics given for continuous variables are median (range), for categorical variables we report n (percent). Box-and-whisker plots of single marker values were used to summarize the distribution of marker values between survivors and non-survivors. For prediction of death within 2 years Cox regression models were used. To illustrate the ability of the different markers for mortality prediction, we calculated Kaplan-Meier survival curves and stratified patients by marker quartiles or combined score tertiles. In addition, time-dependent receiver operating characteristics (ROC) plots were performed. A receiver operating characteristic is a graphical plot of the sensitivity vs. (1 ― specificity), for the outcome (death) as its cut-off is varied. Sensitivity (the proportion of actual positives which are correctly identified as such by a biomarker) and specificity (proportion of negatives which are correctly identified) were calculated for selected cut-off's. Biomarkers were combined with BODE-index parameters by categorization of the biomarkers and allocation of weights to the different categories.

### Results

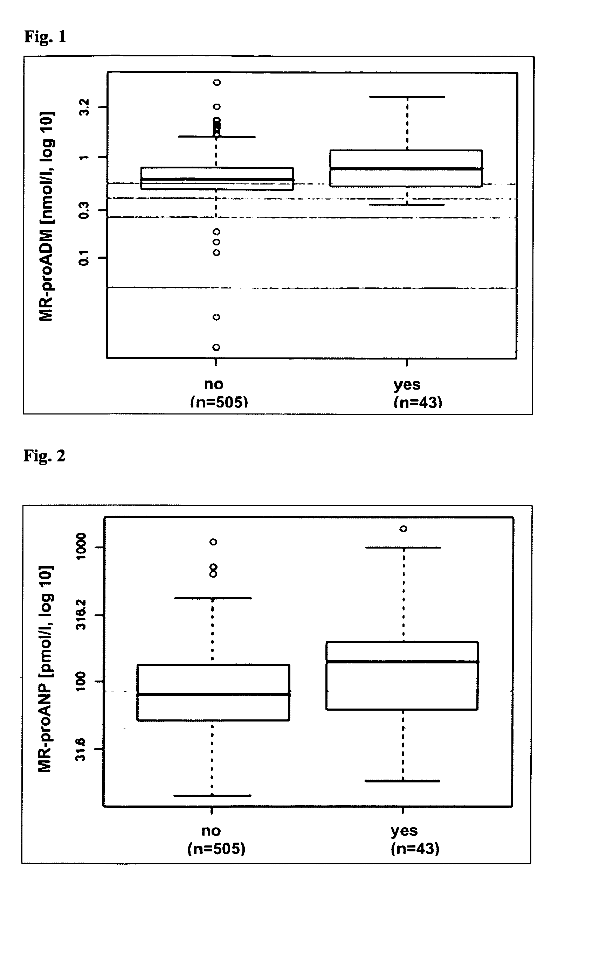

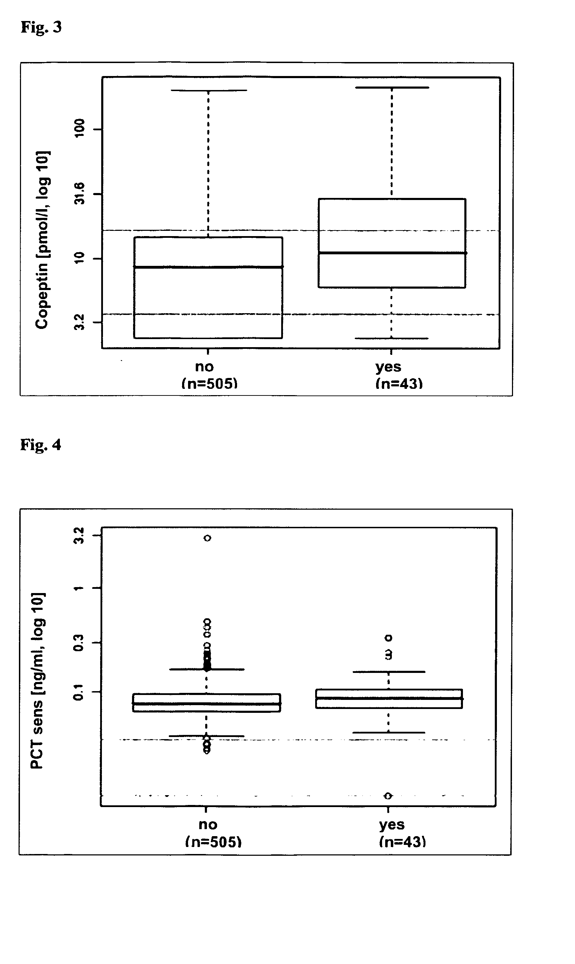

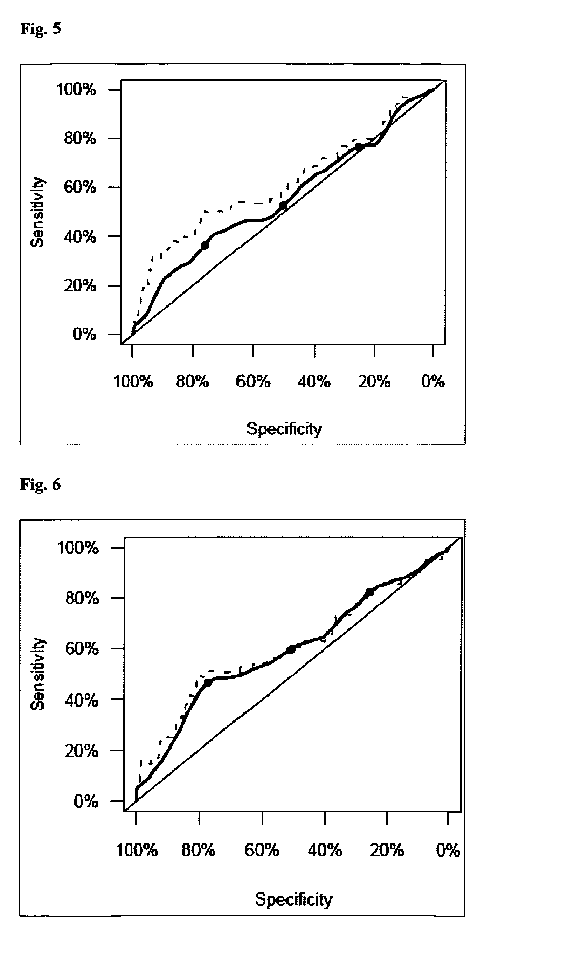

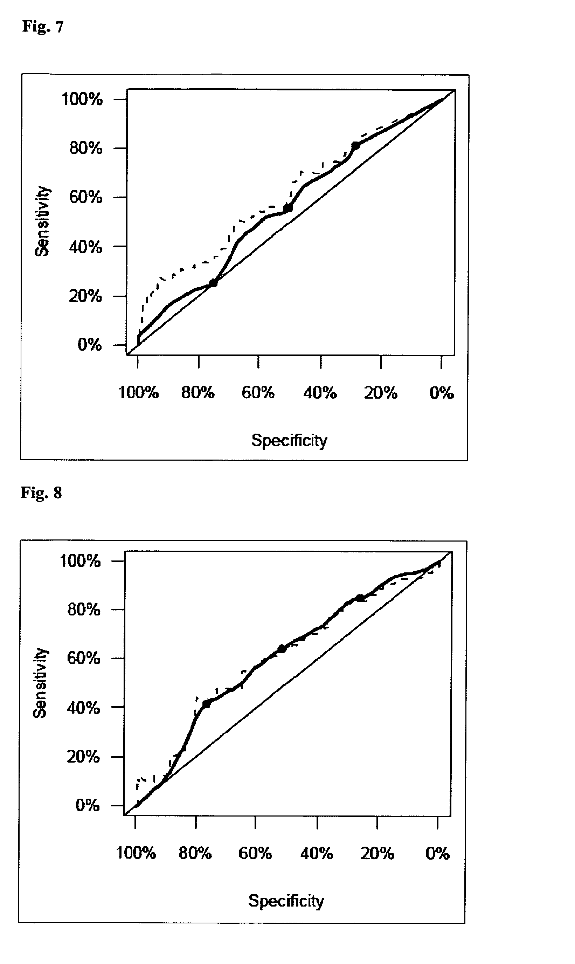

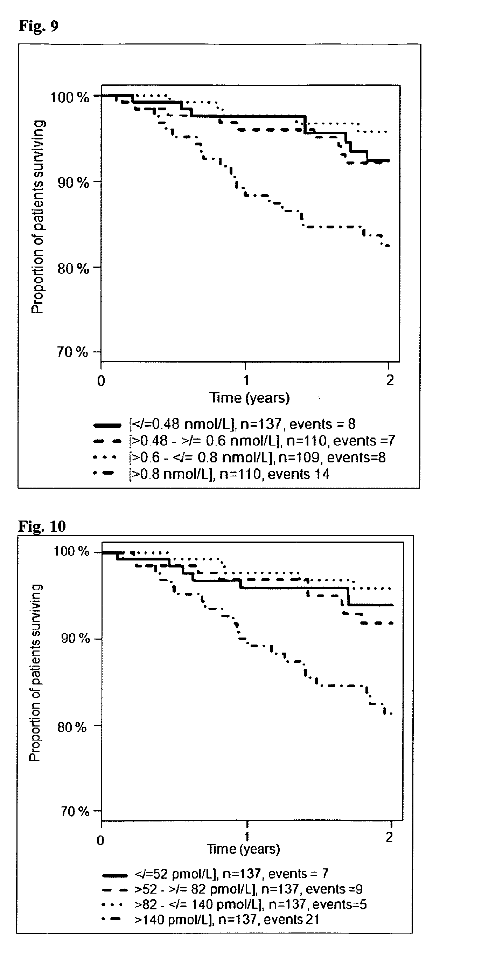

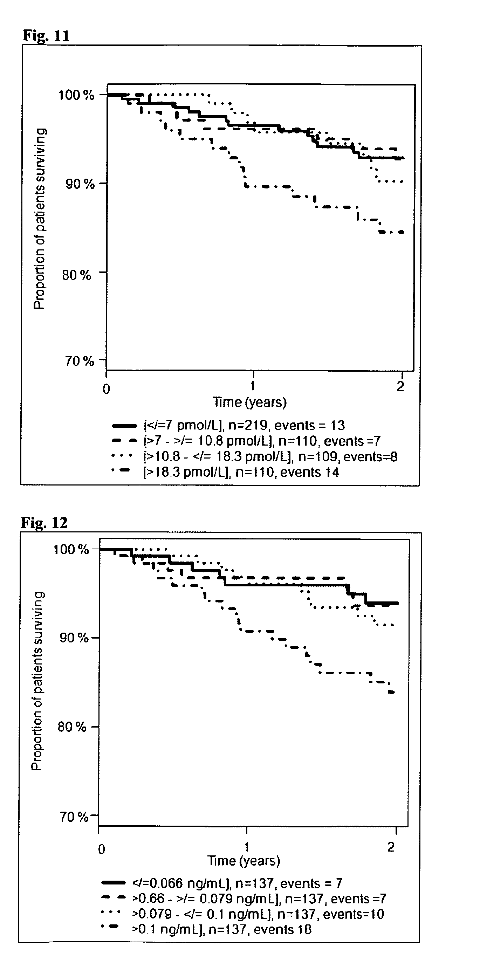

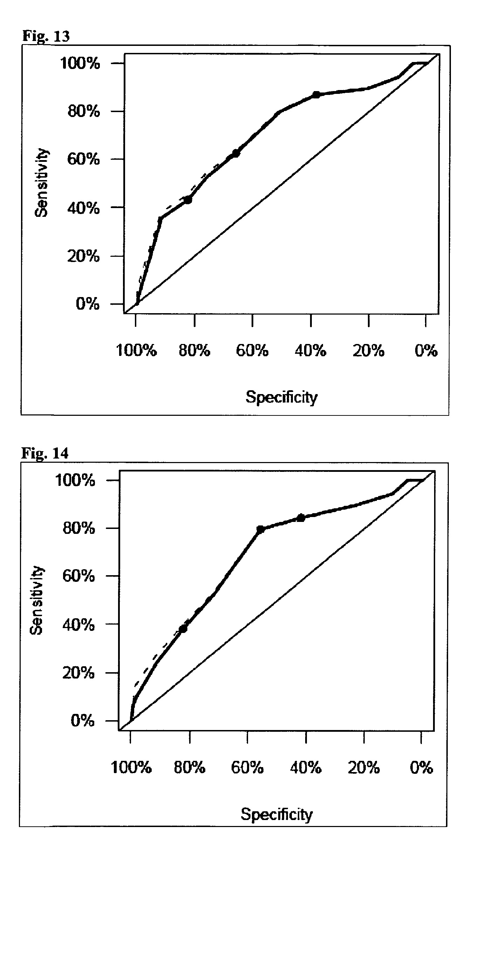

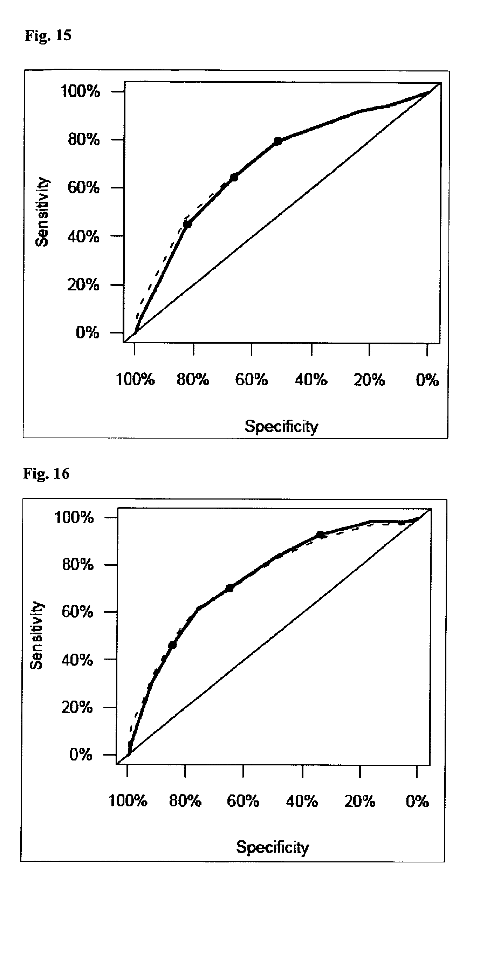

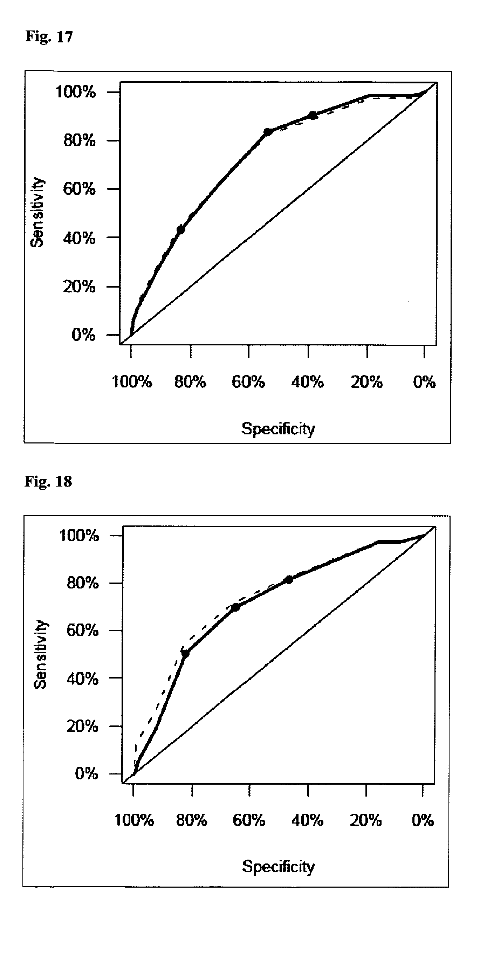

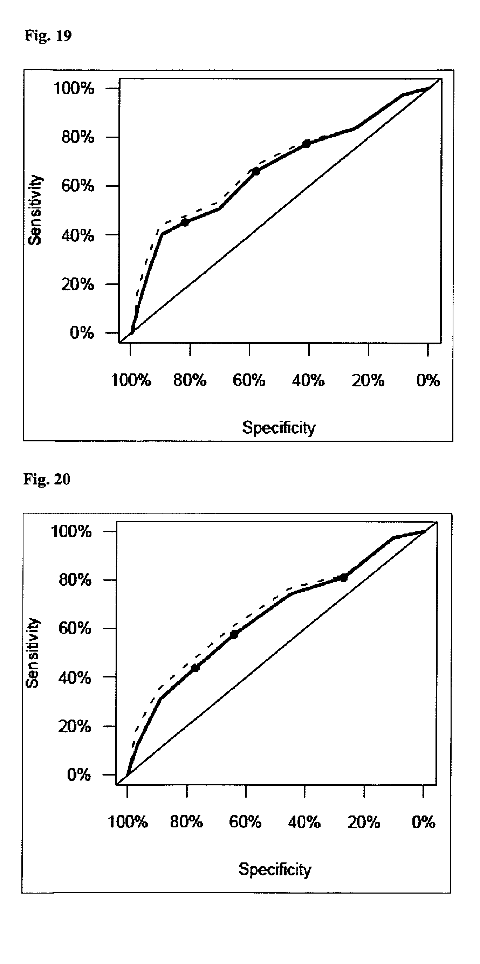

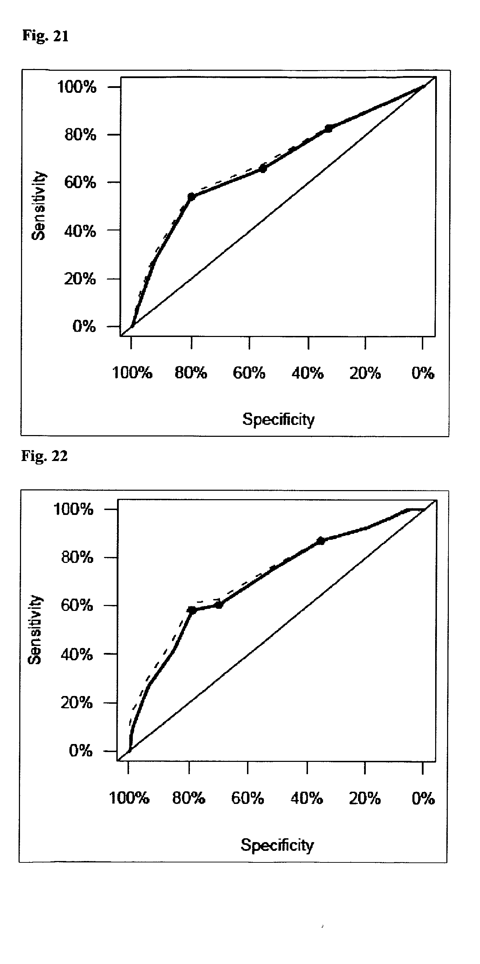

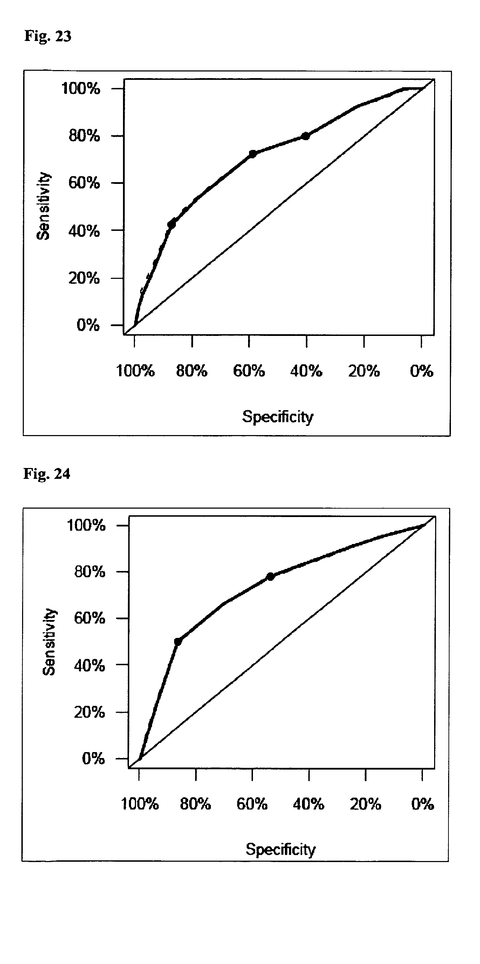

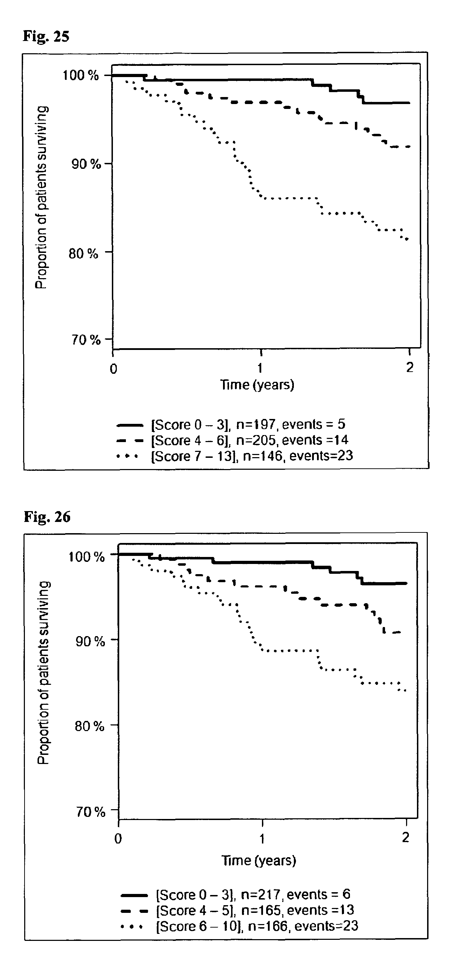

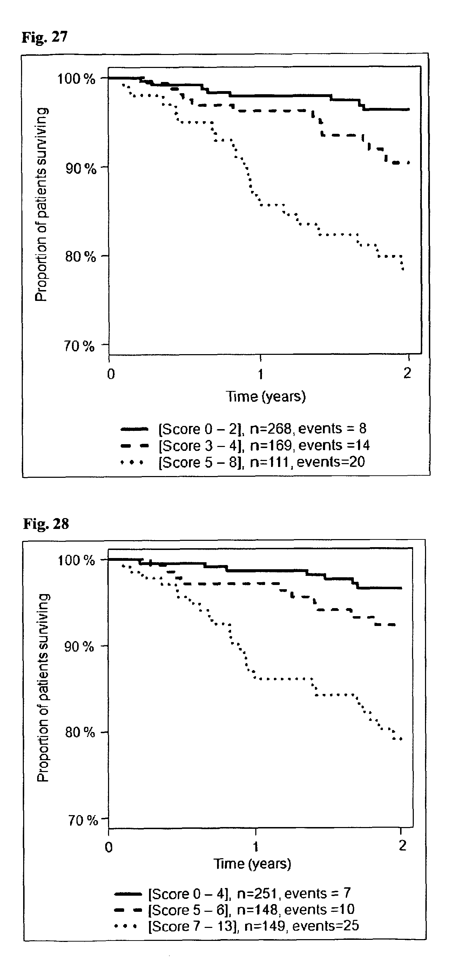

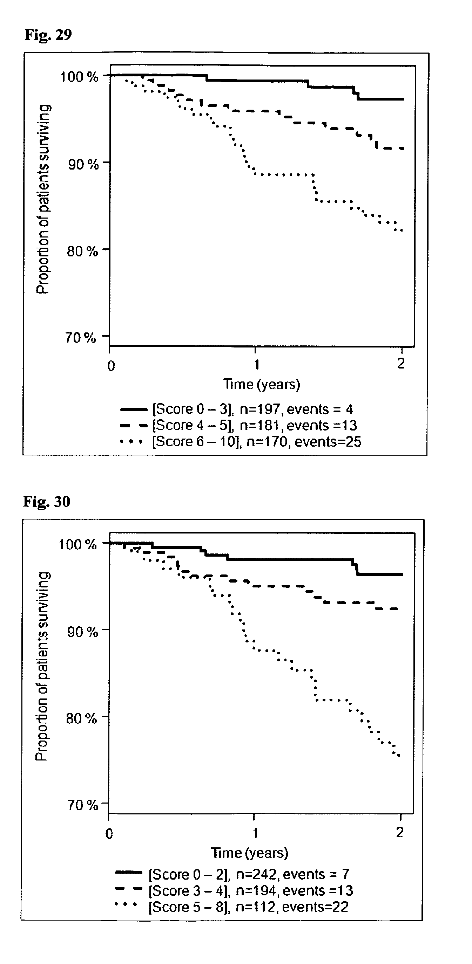

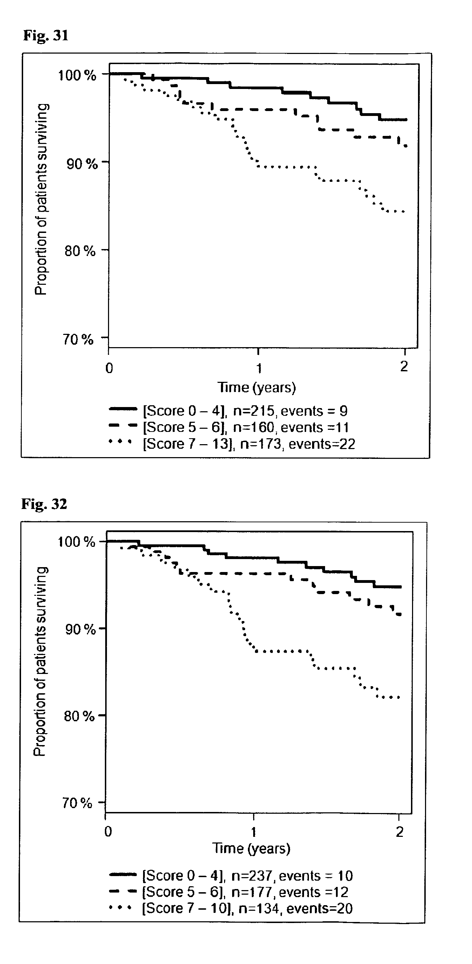

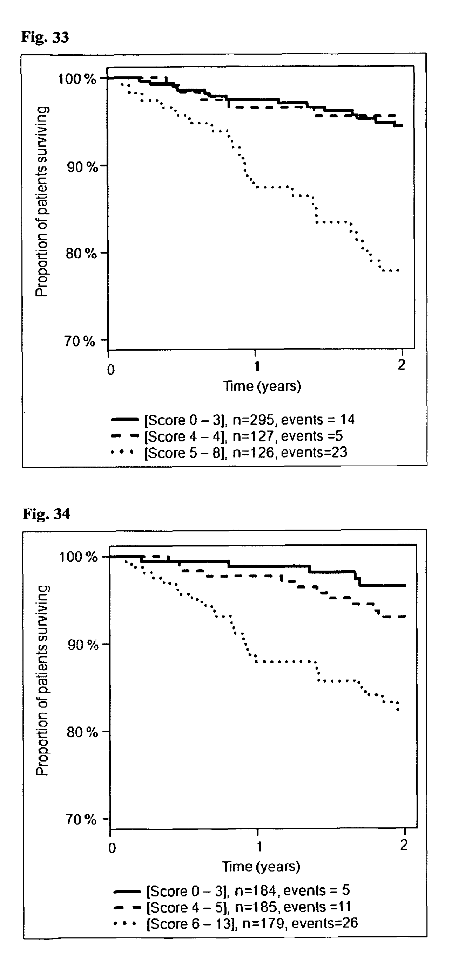

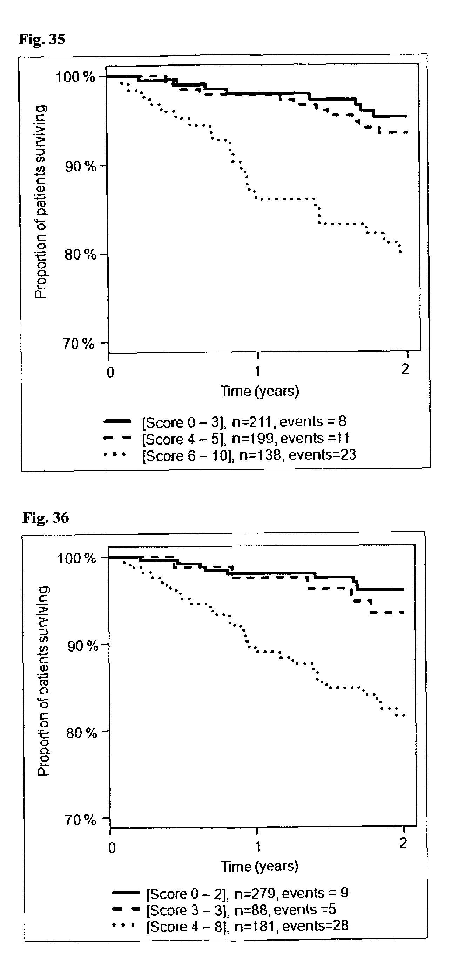

A total of 548 patients were included into the study. Median age was 67 years (range 40 - 88). 166 patients (30.3 %) were female. 43 (7.8%) of these patients died within 2 years. Baseline characteristics of the study population are presented in Table 3.
Box-and-whisker plots of single marker values for the prediction of death within 2 years are shown in Figures 1 to 4. MR-proADM, MR-proANP, Copeptin and PCT concentrations were significantly higher in non-survivors than in survivors, respectively (Kruskal-Wallis test, p < 0.001 for all four marker peptides). Receiver operating characteristics (ROC) for the single markers are shown in Figures 5 to 8. To illustrate the prognostic value of the marker peptides, Kaplan-Meier survival curves were calculated for each single marker, dividing the patients into quartiles depending on the respective marker concentrations (Figures 9 to 12). As shown in Figures 9 to 12, higher mortality rates within 2 years were observed, when MR-proADM, MR-proANP, Copeptin and PCT concentrations, respectively, were in the fourth compared to the first to third quartile.
The overall prognostic accuracy of the marker peptides was assessed using uni- and multivariate Cox regression analyses (Tables 4 and 5). In multivariate models, the biomarkers were combined with the BODE-index parameters as continuous variables (Table 4) and categorized variables (Table 5). When using the biomarkers as categorized variables, two different cut-offs resulting in a Score of 0, 2 or 4 were defined for each biomarker: 0.5 and 0.8 nmol/L for MR-proADM, 50 and 140 pmol/L for MR-proANP, 2 and 14 pmol/L for Copeptin and 0.07 and 0.1 ng/mL for PCT. The respective biomarker score was then added to the score retrieved from the index parameters BODE, BOD or BD to a combined score.
The ROC curves for the biomarker MR-proADM as categorized variable combined with the BODE-, BOD- and BD-index, respectively, are shown in Figures 13 to 15. The ROC curves for the biomarker MR-proANP as categorized variable combined with the BODE-, BOD- and BD-index, respectively, are shown in Figures 16 to 18. The ROC curves for the biomarker Copeptin as categorized variable combined with the BODE-, BOD- and BD-index, respectively, are shown in Figures 19 to 21 and for the biomarker PCT as categorized variable combined with the BODE-, BOD- and BD-index, respectively, in Figures 22 to 24.
These results show that a model including a biomarker (as continuous or categorical variable) was significantly better than the model using the BODE-index parameters alone. Surprisingly, the prediction of death within two years was similar or even better when a biomarker was combined with the index parameters BOD or BD, omitting the index parameters E and/or O. In other words a score using a biomarker in combination with the easily assessable parameters BMI (B) and dyspnea (D), omitting the parameters E (6 minute walking test) and O (degree of airflow obstruction) that are difficult and complex to determine, gives similar or even better results compared to a score using a biomarker in combination with the complete BODE-index.
Kaplan-Meier survival curves were calculated for the combination of marker peptides with the index parameter BODE, BOD or BD (Figures 25 to 36), dividing the patients into risk groups depending on the respective score. For example Figure 25 shows the Kaplan-Meier survival curve for the combination of MR-proADM and the index parameters BODE. The possible score was between 0 and 13 (according to the scoring of BODE in Table 2 plus a score of 0, 2 or 4 for MR-proADM using the cut-off values 0.5 and 0.8 nmol/L as indicated in Table 5). Low mortality rates were observed when the patients had a score between 0 and 3 (2.5%), whereas intermediate mortality rates (6.8%) where observed when the score was between 4 and 6, and high mortality rates (15.8%) where observed at a score of 7 to 13. Figure 27 shows the Kaplan-Meier survival curve for the combination of MR-proADM and the index parameters BD. The possible score (according to the scoring of BD in Table 2 plus a score of 0, 2 or 4 for MR-proADM using the cut-off values 0.5 and 0.8 nmol/L as indicated in Table 5) was between 0 and 8. Low mortality rates were observed when the patients had a score between 0 and 2 (3.0%), whereas intermediate mortality rates (8.3%) where observed when the score was between 3 and 4, and high mortality rates (18.0%) where observed at a score of 5 to 8.

In addition, this finding is emphasized by the following analysis and findings:
Additional 45 patients with a missing 6-minute walking test (index parameter E) were included into the study. 11 of these patients died within a follow-up period of 2 years. The mortality rate for these patients is 24.4% and more than 3-times higher than the mortality rate for the initially analyzed 548 patients where all index parameters (including E) were available. It was therefore hypothesized that the 6-minute walking test in these 45 patients was not missing at random but could rather not be performed due to the poor constitution of the patients. The missing variable E was replaced by the maximum number of 3 points (see Table 2) and the 45 patients were included into the model (Table 6). Again, the model including a biomarker was significantly better than the model using the BODE-index parameters alone, and the prediction of death within two years was similar or even better when a biomarker was combined with the index parameters BOD or BD, omitting the index parameters E and/or O.

### SEQUENCES

SEQ ID NO:1 (amino acid sequence of pre-pro-ANP): 
1 
51 
101 
151 
SEQ ID NO:2 (amino acid sequence of pro-ANP): 
1 NPMYNAVSNA DLMDFKNLLD HLEEKMPLED EVVPPQVLSE PNEEAGAALS
51 PLPEVPPWTG EVSPAQRDGG ALGRGPWDSS DRSALLKSKL RALLTAPRSL
101 RRSSCFGGRM DRIGAQSGLG CNSFRY
SEQ ID NO:3 (amino acid sequence of NT-proANP): 
1 NPMYNAVSNA DLMDFKNLLD HLEEKMPLED EVVPPQVLSE PNEEAGAALS
51 PLPEVPPWTG EVSPAQRDGG ALGRGPWDSS DRSALLKSKL RALLTAPR
SEQ ID NO:4 (amino acid sequence of amino acids 53-90 of pro-ANP): 1 PEVPPWT GEVSPAQRDG GALGRGPWDS SDRSALLKSK L
SEQ ID NO:5 (amino acid sequence of pre-pro-ADM): 
1 MKLVSVALMY LGSLAFLGAD TARLDVASEF RKKWNKWALS RGKRELRMSS
51 SYPTGLADVK AGPAQTLIRP QDMKGASRSP EDSSPDAARI RVKRYRQSMN
101 NFQGLRSFGC RFGTCTVQKL AHQIYQFTDK DKDNVAPRSK ISPQGYGRRR
151 RRSLPEAGPG RTLVSSKPQA HGAPAPPSGS APHFL
SEQ ID NO:6 (amino acid sequence of pro-ADM): 
1 ARLDVASEFR KKWNKWALSR GKRELRMSSS YPTGLADVKA GPAQTLIRPQ
51 DMKGASRSPE DSSPDAARIR VKRYRQSMNN FQGLRSFGCR FGTCTVQKLA
101 HQIYQFTDKD KDNVAPRSKI SPQGYGRRRR RSLPEAGPGR TLVSSKPQAH
151 GAPAPPSGSA PHFL
SEQ ID NO:7 (amino acid sequence of MR-pro-ADM): 1 ELRMSSSYPT GLADVKAGPA QTLIRPQDMK GASRSPEDSS PDAARIRV
SEQ ID NO: 8 (amino acid sequence of pre-pro-AVP): 
1 
51 
101 
151 
SEQ ID NO:9 (amino acid sequence of pro-AVP): 
1     CYFQNCPRGG KRAMSDLELR QCLPCGPGGK GRCFGPSICC ADELGCFVGT
51    AEALRCQEEN YLPSPCQSGQ KACGSGGRCA AFGVCCNDES CVTEPECREG
101   FHRRARASDR SNATQLDGPA GALLLRLVQL AGAPEPFEPA QPDAY
SEQ ID NO:10 (amino acid sequence of CT-pre-proAVP or Copeptin): 1 ASDRSNATQL DGPAGALLLR LVQLAGAPEP FEPAQPDAY
SEQ ID NO:11 (amino acid sequence of Neurophysin II): 
1 AMSDLELRQC LPCGPGGKGR CFGPSICCAD ELGCFVGTAE ALRCQEENYL
51 PSPCQSGQKA CGSGGRCAAF GVCCNDESCV TEPECREGFH RRA
SEQ ID NO:12 (amino acid sequence of PCT): 
1 APFRSALESS PADPATLSED EARLLLAALV QDYVQMKASE LEQEQEREGS
51 SLDSPRSKRC GNLSTCMLGT YTQDFNKFHT FPQTAIGVGA PGKKRDMSSD
101 LERDHRPHVS MPQNAN
SEQ ID NO:13 (amino acid sequence of pre-pro-BNP): 
1 MDPQTAPSRA LLLLLFLHLA FLGGRSHPLG SPGSASDLET SGLQEQRNHL
51 QGKLSELQVE QTSLEPLQES PRPTGVWKSR EVATEGIRGH RKMVLYTLRA
101 PRSPKMVQGS GCFGRKMDRI SSSSGLGCKV LRRH
SEQ ID NO:14 (amino acid sequence of pro-BNP): 
1 HPLGSPGSAS DLETSGLQEQ RNHLQGKLSE LQVEQTSLEP LQESPRPTGV
51 WKSREVATEG IRGHRKMVLY TLRAPRSPKM VQGSGCFGRK MDRISSSSGL
101 GCKVLRRH
SEQ ID NO:15 (amino acid sequence of NT-pro-BNP): 
1 HPLGSPGSAS DLETSGLQEQ RNHLQGKLSE LQVEQTSLEP LQESPRPTGV
51 WKSREVATEG IRGHRKMVLY TLRAPR
SEQ ID NO:16 (amino acid sequence of BNP):

1 SPKMVQGSGC FGRKMDRISS SSGLGCKVLR RH

### TABLES AND FIGURES

**Table 1: Classification of severity of airflow limitation in COPD (Global Initiative for Chronic Obstructive Lung disease, Pocket Guide to COPD diagnosis, management and prevention, revised version 2011) in patients with FEV1/FVC < 0.7**

| Stage | | FEV₁ | |
|---|---|---|---|
| GOLD 1 | Mild | FEV₁ ≥ 80% predicted | At this stage, the patient is probably unaware that lung function is starting to decline. |
| GOLD 2 | Moderate | ≤50% FEV₁ <80% predicted | Symptoms during this stage progress, with shortness of breath developing upon exertion. |
| GOLD 3 | Severe | ≤30% FEV₁ <50% predicted | Shortness of breath becomes worse at this stage and COPD exacerbations are common. |
| GOLD 4 | Very Severe | FEV₁ <30% predicted | Quality of life at this stage is gravely impaired. COPD exacerbations can be life threatening. |

**Table 2: BODE-index**

| **Variable** | **Points on BODE-Index** | | | |
|---|---|---|---|---|
| | **0** | **1** | **2** | **3** |
| **FEV₁ (% of predicted)** | ≥65 | 50-64 | (36-49 | ≤35 |
| **Distance walked in 6 min** | ≥350 | 250-349 | 150-249 | ≤149 |
| **MMRC dyspnea scale** | 0-2 | 3 | 4 | 5 |
| **Body-mass index (kg.m⁻²)** | >21 | ≤21 | | |

**Table 3: Patient characteristics (n = 548 patients)**

| | **Survivors (n=505)** | **Non-Survivors (n=43)** | p |
|---|---|---|---|
| Age | 66.4 (60 ― 73) | 69.1 (62 ― 77) | > 0.05 |
| Gender (female) | 149 (29.5 %) | 17 (39.5 %) | > 0.05 |
| BMI | 26.2 (22.7 ― 28.8) | 24.0 (19.4 ― 27.1) | < 0.05 |
| BODE-index | 3 (1 ― 4) | 5 (2 ― 7) | <0.001 |
| No. exacerbations (per year) | 0.7 (0 ― 1.7) | 0.0 (0 ― 2.3) | > 0.05 |
| MR/proADM (nmol/L) | 0.6 (0.5 ― 0.8) | 0.8 (0.5 ―1.2) | <0.01 |
| MR-proANP (pmol/L) | 80.7 (52.0 ― 135.9) | 141.7 (62.3 ― 197.8) | < 0.05 |
| Copeptin (pmol/L) | 8.6 (2.4 ― 14.5) | 11.1 (6.1 ― 28.8) | < 0.05 |
| PCT (ng/mL) | 0.08 (0.07 ― 0.1) | 0.09 (0.07 ― 0.1) | < 0.05 |

**Table 4: Prediction of mortality within 2 years (biomarker as continuous, BODE-index-parameters as categorical variable) in 548 COPD patients**

| **Universal Model** | **Model** χ**²** | **p-value** | **C-index** |
|---|---|---|---|
| PCT | 4.04 | <0.05 | 0.604 |
| Copeptin | 9.58 | <0.01 | 0.605 |
| MR-proANP | 10.9 | <0.001 | 0.611 |
| MR-proADM | 16.41 | <0.001 | 0.632 |
| BODE | 21.87 | <0.001 | 0.678 |
| BOD | 15.91 | <0.001 | 0.654 |
| BD | 24.15 | <0.001 | 0.683 |

| **Multivariate Model** | **Model** χ**²** | **p-value** | **C-index** |
|---|---|---|---|
| PCT, BODE | 26.67 | <0.001 | 0.698 |
| PCT, BOD | 20.63 | <0.001 | 0.678 |
| PCT, BD | 26.97 | <0.001 | 0.706 |
| Copeptin, BODE | 29.65 | <0.001 | 0.71 |
| Copeptin, BOD | 25.12 | <0.001 | 0.703 |
| Copeptin, BD | 32.65 | <0.001 | 0.724 |
| MR-proANP, BODE | 33.71 | <0.001 | 0.736 |
| MR-proANP, BOD | 29.91 | <0.001 | 0.727 |
| MR-proANP, BD | 36.54 | <0.001 | 0.741 |
| MR-proADM, BODE | 35.28 | <0.001 | 0.75 |
| MR-proADM, BOD | 31.84 | <0.001 | 0.743 |
| MR-proADM, BD | 37.47 | <0.001 | 0.756 |

**Table 5: Prediction of mortality within 2 years (biomarkers and BODE-index parameters as categorical variables) in 548 COPD patients**

| **Multivariate Model** | **Model** χ**²** | **p-value** | **C-index** |
|---|---|---|---|
| PCT, BODE (*) | 33.51 | <0.001 | 0.727 |
| PCT, BOD (*) | 26.48 | <0.001 | 0.713 |
| PCT, BD (*) | 29.18 | <0.001 | 0.726 |
| Copeptin, BODE (**) | 23.37 | <0.001 | 0.687 |
| Copeptin, BOD (**) | 17.81 | <0.001 | 0.673 |
| Copeptin, BD (**) | 22.1 | <0.001 | 0.686 |
| MR-proANP, BODE (~) | 32.29 | <0.001 | 0.739 |
| MR-proANP, BOD (~) | 27.73 | <0.001 | 0.727 |
| MR-proANP, BD (~) | 31.32 | <0.001 | 0.728 |
| MR-proADM, BODE (#) | 27.89 | <0.001 | 0.721 |
| MR-proADM, BOD (#) | 33.43 | <0.001 | 0.706 |
| MR-proADM, BD (#) | 25.38 | <0.001 | 0.715 |

| | | | |
|---|---|---|---|
| Biomarker Cut-offs in this model: * PCT: ≤0.07ng/mL→Score0 ~MR-proANP: ≤50pmol/L→Score0 >0.07 ng/mL and≤ 0.1 ng/mL→ Score 2 >50 pmol/L and ≤ 140 pmol/L→ Score 2 > 0.1 ng/mL→Score 4 > 140 pmol/L → Score 4 ** Copeptin: ≤ 2 pmol/L→Score 0 # MR-proADM: ≤ 0.5 nmol/L→Score 0 >2 pmol/L and ≤ 14 pmol/L→Score 2 >0.5 nmol/L and ≤ 0.8 nmol/L→Score 2 > 14 pmol/L→Score 4 > 0.8 nmol/L → Score 4 | | | |

**Table 6: Prediction of mortality within 2 years (biomarkers and BODE-index parameters as continuous variables) in 593 patients (548 patients plus 45 with missing parameter E which was replaced by the maximum of 3 points)**

| **Univariate Model** | **Model** χ**²** | **p-value** | **C-index** |
|---|---|---|---|
| PCT | 10.78 | 0.0010 | 0.621 |
| Copeptin | 13.64 | 0.0002 | 0.627 |
| MR-proANP | 20.84 | <0.0001 | 0.650 |
| MR-proADM | 26.71 | <0.0001 | 0.661 |
| BODE | 42.69 | <0.001 | 0.718 |
| BOD | 31.95 | <0.0001 | 0.693 |
| BD | 45.06 | <0.0001 | 0.723 |

| **Multivariate Model** | **Model** χ**²** | **p-value** | **C-index** |
|---|---|---|---|
| PCT, BODE | 53.05 | <0.001 | 0.739 |
| PCT, BOD | 43.24 | <0.0001 | 0.726 |
| PCT, BD | 52.15 | <0.0001 | 0.748 |
| Copeptin, BODE | 53.93 | <0.001 | 0.751 |
| Copeptin, BOD | 45.26 | <0.0001 | 0.740 |
| Copeptin, BD | 56.44 | <0.0001 | 0.764 |
| MR-proANP, BODE | 62.04 | <0.001 | 0.772 |
| MR-proANP, BOD | 57.12 | <0.0001 | 0.766 |
| MR-proANP, BD | 66.07 | <0.0001 | 0.782 |
| MR-proADM, BODE | 62.44 | <0.001 | 0.778 |
| MR-proADM, BOD | 56.4 | <0.0001 | 0.774 |
| MR-proADM, BD | 65.15 | <0.0001 | 0.789 |

## Claims

1. A method for the prognosis and/or risk assessment and/or monitoring of therapy and/or management of patients with COPD the method comprising the steps of:
i) providing a sample of a bodily fluid from said patient,
ii) determining in said sample the level of at least one biomarker, selected from the group consisting of pro-adrenomedullin (proADM), pro-natriuretic peptide, pro-Vasopressin (proAVP) and Procalcitonin (PCT) or fragments thereof of at least 12 amino acids in length,
iii) determining one, two or three of the BODE-index parameters body-mass index (BMI, parameter B), degree of airflow obstruction (FEV₁, parameter O), dyspnea (parameter D) and exercise capacity (parameter E),
iv) correlating said level of said at least one biomarker determined in step ii), in combination with said one, two or three BODE-index parameters determined in step iii) to the prognosis and/or risk assessment and/or monitoring of therapy and/or management of patients with COPD.

2. The method according to claim 1, wherein said level of said at least one biomarker or fragments thereof of at least 12 amino acids in length is used in combination with the BODE-index parameters body-mass index (BMI, parameter B), degree of airflow obstruction (FEV₁, parameter O), and dyspnea (parameter D).

3. The method according to claim 1, wherein said level of said at least one biomarker or fragments thereof of at least 12 amino acids in length is used in combination with the BODE-index parameters body-mass index (BMI, parameter B) and dyspnea (parameter D).

4. The method according to any one of claims 1 to 3, wherein said level of said at least one biomarker or fragments thereof of at least 12 amino acids in length is combined as a continuous or categorical variable with said one, two or three BODE-index parameters.

5. The method of claims 1 to 4, wherein said level of said at least one biomarker or fragments thereof of at least 12 amino acids in length and said one, two or three BODE-index parameters are combined in a score.

6. The method of claims 1 to 5, wherein the level of said at least one biomarker or fragments thereof of at least 12 amino acids in length and said one, two or three BODE-index parameters are differently weighted.

7. The method of claim 1 to 6, wherein the patient diagnosed with COPD may be in the stable or unstable (acute exacerbated) state of the disease.

8. The method according to claims 1 to 7, wherein the prognosis and/or risk assessment relates to the risk assessment of mortality and patients are stratified into potential survivors and potential non-survivors.

9. The method of claim 8, wherein the prognosis and/or risk assessment relates to the risk assessment of mortality within 5 years, more preferred within 4 year, even more preferred within 3 years, even more preferred within 2 years, even more preferred within 1 year, most preferred within 6 months.

10. The method according to claims 1 to 7, wherein the prognosis and/or risk assessment relates to the risk of getting an acute exacerbation and patients are stratified into either a group of patients likely getting an acute exacerbation or into a group of patients which do not likely get an acute exacerbation.

11. The method of claim 10, wherein the prognosis and/or risk assessment relates to the risk assessment of getting an acute exacerbation within 2 years, more preferred within 1 year, even more preferred within 6 months, even more preferred within 3 months, even more preferred within 90 days, even more preferred within 1 month, even more preferred within 30 days, even more preferred within 14 days, most preferred within 7 days.

12. The method according to claims 1 to 10, wherein the sample is selected from the group comprising a blood sample, a serum sample, a plasma sample, a cerebrospinal fluid sample, a saliva sample and a urine sample or an extract of any of the aforementioned samples.

13. The method according to any of the preceding claims, wherein the level of a precursor fragment, selected from the group consisting of MR-proADM, NT-proBNP, BNP, MR-proANP, Copeptin, PCT 1-116, PCT 2-116 or PCT 3-116, is determined.

14. The method according to claim 13, wherein the cut-off values of MR-proADM are between 0.5 and 2 nmol/L, more preferred between 0.5 and 1 nmol/L, most preferred between 0.5 and 0.8 nmol/L.

15. The method according to claim 13, wherein the cut-off values for MR-proANP are between 50 and 250 pmol/L, more preferred between 50 and 200 pmol/L, most preferred between 50 and 140 pmol/L.

16. The method according to claim 13, wherein the cut-off values for Copeptin are between 2 and 30 pmol/L, more preferred between 2 and 20 pmol/L, most preferred between 2 and 14 pmol/L.

17. The method according to claim 13, wherein the cut-off values for PCT are between 0.07 and 0.5 ng/mL, more preferred between 0.07 and 0.25 ng/mL, even more preferred between 0.07 and 0.2 ng/mL, most preferred between 0.07 and 0.1 ng/mL.
